# EUROPÄISCHE PATENTSCHRIFT

(11) **EP 3 134 027 B1**
(45) Veröffentlichungstag und Bekanntmachung des Hinweises auf die Patenterteilung: **02.09.2020**
(21) Anmeldenummer: 15723436.0
(22) Anmeldetag: 23.04.2015
(51) Int. Cl.: A61C 9/00

(54) **VERFAHREN ZUR DURCHFÜHRUNG EINER OPTISCHEN DREIDIMENSIONALEN AUFNAHME**
METHOD FOR PERFORMING AN OPTICAL THREE-DIMENSIONAL RECORDING
PROCÉDÉ DE RÉALISATION D'UN CLICHÉ OPTIQUE TRIDIMENSIONNEL

(30) Priorität: 23.04.2014 DE 102014207667
(43) Veröffentlichungstag der Anmeldung: 01.03.2017
(73) Patentinhaber: Sirona Dental Systems GmbH, 64625 Bensheim (DE)
(72) Erfinder: ADAMSON, Anders, 64297 Darmstadt (DE); BOBACH, Tom, 64625 Bensheim (DE); GRUND, Nico, 64646 Heppenheim (DE)
(74) Vertreter: Özer, Alpdeniz
(86) Internationale Anmeldenummer: PCT/EP2015/058767
(87) Internationale Veröffentlichungsnummer: WO 2015/162199

(56) Entgegenhaltungen:
- WO-A1-2014/027026

## Beschreibung

### Technisches Gebiet

Die Erfindung betrifft ein Verfahren zur Durchführung einer optischen dreidimensionalen Aufnahme unter Verwendung einer handgehaltenen dentalen Kamera, wobei die Kamera während der Vermessung nacheinander mehrere einzelne optische Aufnahmen aufnimmt. Die einzelnen dreidimensionalen optischen Aufnahmen werden dann zu einer dreidimensionalen Gesamtaufnahme eines zu vermessenden dentalen Objekts durch Registrierung zusammengefügt.

### Stand der Technik

Aus dem Stand der Technik sind mehrere Registrierungsverfahren bekannt, bei denen mehrere optische Einzelaufnahmen durchgeführt werden und anschließend eine Registrierung erfolgt.

WO 2014/027026 A1 offenbart ein Verfahren zur Registrierung von einzelnen dreidimensionalen optischen Aufnahmen zu einer Gesamtaufnahme eines zu vermessenden dentalen Objekts, wobei unter Verwendung eines Computers automatisch überprüft, ob ein Überlappungsbereich zwischen den zusammenzufügenden Aufnahmen bestimmte Registrierungsbedingungen für eine fehlerfreie Registrierung erfüllt. Falls der Überlappungsbereich die Registrierungsbedingungen erfüllt, wird die Registrierung zwischen den zusammenzufügenden Aufnahmen durchgeführt und eine erste Aufnahmesequenz wird fortgesetzt, wobei die Aufnahmen der ersten Aufnahmesequenz anschließend zu einem ersten Cluster zusammengefügt werden.

Bei der Registrierung werden übereinstimmende Bereiche, sogenannte Überlappungsbereiche, erkannt und zueinander registriert, sodass aus den einzelnen optischen Aufnahmen eine Gesamtaufnahme gebildet wird.

Bei dieser so genannten On-The-Fly-Vermessung kann es zu einer fehlerhaften Registrierung kommen. Dies kann beispielsweise durch zu kleine Überlappungsbereiche, durch Aufnahmefehler oder durch störende Objekte in den Einzelaufnahmen, wie Wange oder Zunge, verursacht werden.

Ein Nachteil dieses Verfahrens besteht darin, dass während der manuellen Vermessung die handgehaltene Dentalkamera durch den Benutzer frei ohne eine Benutzerführung bewegt wird. Dadurch kann es also dazu kommen, dass manche Bereiche des Objekts unnötigerweise mehrfach vermessen werden und dadurch das zu registrierende Datenvolumen und die Aufnahmezeit steigen. Während der Vermessung kann es auch dazu kommen, dass manche Bereiche des Objekts ausgelassen werden und dadurch Lücken entstehen. Diese Lücken müssen dann anschließend durch zusätzliche Aufnahmensequenzen geschlossen werden.

Die Aufgabe der vorliegenden Erfindung besteht daher darin, ein Verfahren bereitzustellen, das eine zuverlässige, vollständige und schnelle Vermessung des Objekts mit einer wiederholbaren Registrierungssituation ermöglicht.

### Darstellung der Erfindung

Die Erfindung betrifft ein Verfahren zur Durchführung einer optischen dreidimensionalen Aufnahme unter Verwendung einer handgehaltenen dentalen Kamera, wobei die Kamera während der Vermessung mehrere einzelne dreidimensionale optische Aufnahmen nacheinander mit einer festgelegten Frequenz automatisch aufnimmt. Die einzelnen dreidimensionalen optischen Aufnahmen werden dann durch ein Registrierungsverfahren zu einer Gesamtaufnahme eines zu vermessenden dentalen Objekts zusammengefügt. Vor der Durchführung der Vermessung wird ein dreidimensionales Standardkiefermodell mittels einer Anzeigevorrichtung angezeigt, wobei ein erster Kontrollpunkt am Standardkiefermodell mittels der Anzeigevorrichtung angezeigt wird. Dabei wird die handgehaltene Kamera relativ zum aufzunehmenden Objekt so positioniert, dass die Kamera auf den ersten Kontrollpunkt des Standardkiefermodells zeigt und einen dem ersten Kontrollpunkt entsprechenden Aufnahmebereich des dentalen Objekts in einer optischen Aufnahme aufnimmt.

Die Kamera nimmt also während der Vermessung mehrere einzelne Aufnahmen beispielsweise mit einer Frequenz von 18 Hz automatisch auf, wobei die handgehaltenen Kamera relativ zum Objekt bewegt wird. Die einzelnen Aufnahmen müssen also nicht manuell ausgelöst werden.

Die optischen Aufnahmen werden mittels der dentalen Kamera vermessen, die beispielsweise nach einem Streifenprojektionsverfahren funktionieren kann. Bei dem Streifenprojektionsverfahren können die einzelnen auf das Objekt projizierten Streifen anhand der Intensität, Farbe, Polarisation, Kohärenz, Phase, Kontrast, Ort oder Laufzeit identifiziert werden. Anschließend werden unter Verwendung eines Triangulationsverfahrens die 3D-Koordinaten der einzelnen Messpunkte auf dem Objekt berechnet. Bei der Kodierung durch die Farbe kann anhand einer bestimmten Reihenfolge der Farbstreifen jedes der Farbstreifen eindeutig identifiziert werden. Zur Erzeugung des Streifenmusters kann beispielsweise ein Dia bzw. ein Gitter verwendet werden.

Alternativ kann auch ein konfokales Vermessungsverfahren verwendet werden, um die einzelnen dreidimensionalen Aufnahmen des Objekts aufzunehmen.

Während der Vermessung wird die handgehaltene Dentalkamera relativ zum dentalen Objekt, wie einem Unterkiefer oder einem Oberkiefer, bewegt, wobei in regelmäßigen Zeitabständen die dreidimensionalen optischen Aufnahmen erzeugt werden. Die einzelnen Aufnahmen können beispielsweise mit einer Taktfrequenz zwischen 10 Hz und 20 Hz erzeugt werden. Die Registrierung erfolgt mittels eines Computers, der die aufgenommenen Aufnahmen auswertet. Als Registrierungsverfahren kann beispielsweise das ICP-Registrierungsverfahren (Iterative-Closest-Point-Algorithmus) verwendet werden. Dieser Algorithmus ist ein bekanntes Verfahren zur Registrierung von zweidimensionalen bzw. dreidimensionalen Objekten. Das Ziel dieses Verfahrens ist es, zwei unterschiedliche 3D-Modelle eines Objekts distanzminimierend zueinander auszurichten. Dazu werden unterschiedliche Rotationen und Translationen auf korrespondierende Punktepaare der beiden zu registrierenden Aufnahmen angewendet und dabei ein quadratischer Fehler der Abstände zwischen den Punktepaaren minimiert. Diese iterative Annäherung wird solange ausgeführt, bis die beiden Aufnahmen innerhalb des Überlappungsbereichs übereinstimmen.

Alternativ oder ergänzend kann die Registrierung auch aufgrund der Farbe des aufgenommenen Objekts, der Oberflächenkrümmung des aufgenommenen Objekts oder aufgrund von charakteristischer Geometrien des Objekts erfolgen. Bei der Registrierung aufgrund charakteristischer Geometrien wird ein Mustererkennungsalgorithmus verwendet, bei dem die beiden zu registrierenden Aufnahmen nach einem bestimmten geometrischen Muster, wie nach einer Okklusionsfläche eines bestimmten Zahns, durchsucht werden.

Das Registrierungsverfahren kann zu Registrierungsfehlern führen, falls beispielsweise die Kamera in Relation zum Objekt zu schnell bewegt wird und dadurch die Größe des Überlappungsbereichs unzureichend ist. Ein weiterer Grund könnte sein, dass ein Autofokus der dentalen Kamera unscharf eingestellt ist und dadurch das Objekt undeutlich abgebildet wird, so dass die Aufnahmequalität der Aufnahme nicht ausreichend ist. Ein weiterer Grund könnte sein, dass bewegliche Objekte, wie Zunge des Patienten oder Finger des behandelnden Zahnarztes, während der Vermessung erfasst werden. Dies führt dazu, dass die Überlappungsbereiche der Aufnahmen nicht übereinstimmen.

Bei diesem Verfahren erfolgt vor der Durchführung der Vermessung eine Benutzerführung, wobei am dreidimensionalen Standardkiefermodell, das in einem Speicher eines Computers abgespeichert sein kann, mittels einer Anzeigevorrichtung der erste Kontrollpunkt angezeigt wird.

Die Anzeigevorrichtung kann beispielsweise ein Monitor sein, der an einen Computer angeschlossen ist. Das Standardkiefermodell kann beispielsweise halbtransparent angezeigt werden, wobei die einzelnen Aufnahmen sowie die registrierte Gesamtaufnahme als ein undurchsichtiges dreidimensionales Modell in Überlagerung mit dem Standardkiefermodell dargestellt werden können. Dadurch kann der Benutzer auf eine einfache Art und Weise visuell erkennen, welche Bereiche des Kiefers bereits vermessen wurden.

Das dentale Objekt kann auch ein einzelner Zahn, eine Gruppe von Zähnen oder auch eine Präparation für ein einzusetzendes Zahnersatzteil sein.

Dadurch kann also der erste Kontrollpunkt als Benutzerführung im Bereich der Präparation angeordnet sein, um diese vollständig vermessen.

Das Standardkiefermodell kann dabei abhängig von Patientendaten modifiziert sein. Falls beispielsweise bestimmte Zähne der zu vermessenden Kiefer eines Patienten, wie die hinteren Backenzähne mit den FDI-Nummern 18, 28, 38 oder 48 fehlen, fehlen diese Zähne auch im Standardkiefermodell. Falls aus den Patientendaten bekannt ist, dass ein bestimmter Zahn repariert wurde, kann das Standardkiefermodell auch eine standardisierte Präparation aufweisen. Dadurch kann sich der Benutzer in der zu vermessenden Zahnsituation einfacher orientieren.

Zur Benutzerführung wird also der erste Kontrollpunkt am Standardkiefermodell angezeigt, so dass der Benutzer erkennt in welchem Bereich des Kiefers er mit der Vermessung beginnen soll.

Der Kontrollpunkt kann beispielsweise farblich oder graphisch hervorgehoben werden.

Der Kontrollpunkt kann beliebig, wie beispielsweise als ein Punkt, als ein roter Kreis oder auch als ein Kreuz, dargestellt werden.

Ein Vorteil dieses Verfahrens besteht darin, dass die Benutzerführung mittels der Anzeigevorrichtung am Standardkiefermodell eine wiederholbare Vermessung des Objekts und eine zuverlässige Registrierung ermöglicht, da das Objekt auf dieselbe Art und Weise den Kontrollpunkten folgend entlang der angezeigten Aufnahmepfade vermessen werden kann.

Ein weiterer Vorteil dieses Verfahrens besteht darin, dass im Gegensatz zu einer On-The-Fly-Vermessung das Objekt nicht beliebig sondern entlang der optimierten Aufnahmepfade vermessen wird, so dass Aufnahmezeit und Datenvolumen minimiert werden.

Ein weiterer Vorteil dieses Verfahrens besteht darin, dass eine zuverlässige globale Registrierung ermöglicht wird, da durch die optimierten Aufnahmepfade alle Cluster in einem stabilen Gerüst zueinander registriert werden.

Vorteilhafterweise kann die Position des ersten Kontrollpunktes durch eine Handlung eines Benutzers bestätigt werden.

Dadurch kann der Benutzer die Position des ersten Kontrollpunktes bestätigen, ohne die Kamera aus der Hand zu legen.

Vorteilhafterweise kann der Benutzer die dentale Kamera relativ zum Objekt am ersten Kontrollpunkt für eine festgelegte Zeitdauer still halten bis ein akustisches, visuelles und/oder haptisches Signal als Rückmeldung erfolgt.

Dadurch kann die dentale Kamera über dem Kontrollpunkt, beispielsweise für eine Zeitdauer von 2 Sekunden, stillgehalten werden. Als Rückmeldung kann ein akustisches, visuelles und/oder haptisches Signal, beispielsweise mittels einer LED an der Kamera, durch Vibration oder durch einen Piepston, erfolgen. Dadurch wird dem Benutzer mitgeteilt, dass der erste Kontrollpunkt bestätigt wurde.

Die Geschwindigkeit bzw. die Beschleunigung der Kamera relativ zum Objekt kann unter Verwendung von Beschleunigungssensoren in der Kamera oder auch durch die Auswertung der einzelnen Aufnahmen bestimmt werden. Dabei wird die Veränderung von charakteristischen Strukturen in den Aufnahmen bestimmt und daraus die Geschwindigkeit ermittelt.

Das Signal als Rückmeldung kann beispielsweise erfolgen, wenn die Beschleunigung bzw. die Geschwindigkeit der Kamera relativ zum Objekt einen bestimmten Schwellenwert für eine bestimmte Dauer nicht überschreitet.

Vorteilhafterweise kann der Benutzer den ersten Kontrollpunkt durch das Betätigen einer Taste an der Kamera bestätigen.

Dadurch kann der Benutzer den ersten Kontrollpunkt bestätigen, ohne die Hand von der Kamera zu nehmen.

Vorteilhafterweise kann der Benutzer den ersten Kontrollpunkt durch das Ausführen einer Geste mit der Kamera bestätigen.

Eine Geste könnte beispielsweise einfaches oder zweifaches Winken der handgehaltenen Kamera nach rechts sein. Dies wird dann als ein Befehl interpretiert, den ersten Kontrollpunkt zu bestätigen und entsprechend in der Software an der Anzeigevorrichtung zu markieren.

Vorteilhafterweise kann der Benutzer den ersten Kontrollpunkt durch einen Sprachbefehl bestätigen, indem der Sprachbefehl in einer Audioaufnahme aufgezeichnet wird und mittels einer Spracherkennung erkannt wird.

Dadurch kann also der Benutzer durch das Aussprechen des jeweiligen Sprachbefehls den Kontrollpunkt bestätigen.

Vorteilhafterweise kann der Benutzer den ersten Kontrollpunkt durch das Betätigen eines Eingabemittels, wie einer Maus oder eine Tastatur, bestätigen.

Dadurch kann also der Benutzer mittels der Eingabemittel, die am Computer angeschlossen sind, den Kontrollpunkt bestätigen.

Vorteilhafterweise kann das dentale Objekt ein gesamter Oberkiefer und/oder ein Unterkiefer sein.

Das dentale Objekt kann also der gesamte Oberkiefer, der gesamte Unterkiefer oder auch ein einzelner Zahn, eine Gruppe von Zähnen oder eine Präparation sein.

Vorteilhafterweise kann für die Vermessung eines gesamten Ober- oder Unterkiefers der erste Kontrollpunkt in der Mitte einer Okklusalfläche eines Backenzahns angeordnet sein, der sich an einem linken Ende oder an einem rechten Ende des Standardkiefermodells befindet.

Dadurch kann also der erste Kontrollpunkt beispielsweise im Zentrum der Okklusalfläche eines Backenzahns 18 oben rechts, eines Backenzahns 28 oben rechts für den Oberkiefer oder eines Backenzahns 48 unten rechts bzw. eines Backenzahns 38 unten links eines Unterkiefers nach dem FDI-Zahnschema sein. Dieser Kontrollpunkt ist also bereits vor der Vermessung am Standardkiefermodell festgelegt.

Vorteilhafterweise kann der Benutzer die Lage des festgelegten ersten Kontrollpunktes unter Verwendung eines Eingabemittels relativ zum Standardkiefermodell verschieben und damit neu festlegen.

Dadurch kann also der Benutzer mittels eines Eingabemittels, wie einer Maus den ersten Kontrollpunkt verschieben und an die tatsächliche Zahnsituation anpassen. Beispielsweise falls die hinteren Backenzähne 18, 28, 48 oder 38 nach dem FDI-Zahnschema fehlen. Ein weiterer Grund für die Anpassung könnten abweichende Abmessungen des tatsächlichen Kiefers vom Standardkiefermodell sein.

Vorteilhafterweise kann zusätzlich zum ersten Kontrollpunkt an einem gegenüberliegenden Ende des zu vermessenden Kiefers ein zweiter Kontrollpunkt festgelegt und mittels der Anzeigevorrichtung angezeigt werden, wobei zwischen dem ersten Kontrollpunkt und dem zweiten Kontrollpunkt ein erster Aufnahmepfad festgelegt wird, der mittels der Anzeigevorrichtung am Standardkiefermodell angezeigt wird.

Dadurch wird also zwischen einem ersten Kontrollpunkt am Backenzahn 18 bzw. 48 und einem zweiten Kontrollpunkt am Backenzahn 28 bzw. 38 ein Aufnahmepfad festgelegt, der beispielsweise durch die Zahnzentren der einzelnen Zähne des Standardkiefermodells verlaufen kann. Die Zahnzentren sind dabei als Zentren der Okklusalflächen der Backenzähne bzw. als Mitte der Inzisalkanten der Schneidezähne definiert. Dieser Aufnahmepfad entspricht dann der optimalen Vermessungsbewegung der Kamera für die okklusale Vermessung. Der graphisch angezeigte Aufnahmepfad dient als eine Benutzerführung für den Benutzer während der Vermessung.

Vorteilhafterweise kann eine okklusale Vermessung aus einer okklusalen Richtung relativ zum Kiefer durchgeführt werden, wobei die manuell gehaltene Dentalkamera entlang des angezeigten ersten Aufnahmepfads bewegt wird bis der zweite Kontrollpunkt erreicht ist.

Dadurch wird der Kiefer also aus okklusaler Richtung vermessen.

Vorteilhafterweise kann die Position des zweiten Kontrollpunktes durch eine Handlung eines Benutzers bestätigt werden.

Dadurch kann auch der zweite Kontrollpunkt durch eine der oben beschriebenen Handlungen, wie das Stillhalten der Kamera oder durch Betätigen einer Taste an der Kamera, bestätigt werden.

Vorteilhafterweise kann eine linguale beziehungsweise eine palatinale Vermessung von in oraler Richtung liegenden Zahnflächen des Oberkiefers beziehungsweise des Unterkiefers als Objekt erfolgen. Dabei wird ein dritter Kontrollpunkt am gegenüberliegenden Ende des Kiefers ausgehend vom zweiten Kontrollpunkt festgelegt, wobei ein zweiter Aufnahmepfad zwischen dem zweiten Kontrollpunkt und dem dritten Kontrollpunkt festgelegt und mittels der Anzeigevorrichtung angezeigt wird. Anschließend wird die handgehaltene Kamera während der Vermessung entlang des zweiten Aufnahmepfads bewegt, wobei die Position des dritten Aufnahmepunktes durch eine Handlung des Benutzers bestätigt wird. Der dritte Kontrollpunkt kann dabei die gleiche Position wie der erste Kontrollpunkt aufweisen.

Dadurch wird also eine linguale bzw. palatinale Vermessung des Oberkiefers bzw. des Unterkiefers entlang des zweiten Aufnahmepfads als Benutzerführung durchgeführt.

Vorteilhafterweise kann eine bukkale Vermessung aus einer bukkalen Richtung durchgeführt werden, wobei in einem ersten Schritt eine erste bukkale Vermessung ausgehend von einem vierten Kontrollpunkt an einem ersten Ende des Kieferbogens zur Mitte des Kieferbogens und darüber hinaus bis zu einem fünften Kontrollpunkt entlang eines dritten Aufnahmepfades durchgeführt wird, wobei anschließend in einem zweiten Schritt eine zweite bukkale Vermessung ausgehend von einem sechsten Kontrollpunkt an einem zweiten gegenüberliegenden Ende des Kieferbogens zur Mitte des Kieferbogens und darüber hinaus bis zu einem siebten Kontrollpunkt entlang eines vierten Aufnahmepfades durchgeführt wird, der mittels der Anzeigevorrichtung angezeigt wird.

Dadurch wird also eine bukkale Vermessung des Oberkiefers oder des Unterkiefers entlang des dritten und des vierten Aufnahmepfades als Benutzerführung durchgeführt.

Vorteilhafterweise können ein erstes Cluster aus der ersten bukkalen Vermessung und ein zweites Cluster aus der zweiten bukkalen Vermessung unter Verwendung eines gemeinsamen Überlappungsbereichs in der Mitte des Kieferbogens zueinander registriert werden.

Dadurch werden die beiden Cluster zu der Gesamtaufnahme des gesamten Kieferbogens registriert.

Vorteilhafterweise kann mindestens eine Streifen-Aufnahmesequenz in bukkaler Richtung senkrecht zu einer Kieferkurve des zu vermessenden Kieferbogens entlang eines fünften Aufnahmepfades, der vor der Vermessung dieser Streifen- Aufnahmesequenz mittels der Anzeigevorrichtung angezeigt wird, durchgeführt werden. Dabei werden die bereits erzeugten Cluster aus der okklusalen Vermessung, der lingualen Vermessung und/oder der bukkalen Vermessung miteinander verknüpft und dadurch die Registrierung verbessert.

Durch die Vermessung der Streifen-Aufnahmesequenz werden also die Bilddaten der Cluster aus der Okklusalvermessung, der lingualen Vermessung und/oder der bukkalen Vermessung miteinander verknüpft. Zur Verbesserung der Registrierung können auch zwei oder drei Streifen-Aufnahmesequenzen beispielsweise oben rechts, mitte, oben links für den Oberkiefer oder unten rechts, mitte, unten links für den Unterkiefer durchgeführt werden.

Vorteilhafterweise kann eine Aufbissregistrierung durchgeführt werden, wobei ein erstes dreidimensionales Modell des Oberkiefers relativ zu einem zweiten dreidimensionalen Modell des Unterkiefers registriert wird. Dabei wird eine bukkale Aufnahmesequenz in einer Aufbissstellung entlang eines siebten Aufnahmepfades vermessen, der mittels der Anzeigevorrichtung zwischen entsprechenden Kontrollpunkten angezeigt wird.

Dadurch wird also eine Aufbissregistrierung durchgeführt, wobei der siebte Aufnahmepfad, der mittels der Anzeigevorrichtung dargestellt wird, als eine Benutzerführung dient. Bei der Aufbissregistrierung können auch die ermittelten Kieferkurven des Oberkiefers und des Unterkiefers verwendet werden, indem die beiden Kieferkurven parallel zueinander ausgerichtet werden. Anschließend wird dann die bukkale Aufnahmesequenz verwendet, um eine feinere Registrierung des Oberkiefers zum Unterkiefer durchzuführen.

Vorteilhafterweise kann ein tatsächlicher Aufnahmepfad der Kamera ermittelt werden, der durch die Projektionen der Zentren der einzelnen Aufnahmen entlang einer Kameraachse auf einer Oberfläche des Objekts gebildet wird, wobei der tatsächliche Aufnahmepfad der Kamera mittels der Anzeigevorrichtung angezeigt wird.

Dabei kann während der Vermessung eine Abweichung zwischen den tatsächlichen Aufnahmepfad und dem geplanten Aufnahmepfad ermittelt werden.

Falls diese Abweichung einen festgelegten Schwellenwert überschreitet, kann eine Fehlermeldung mittels der Anzeigevorrichtung angezeigt werden. Daraufhin kann der Benutzer aufgefordert werden die Vermessung an einem neuen Kontrollpunkt fortzusetzen.

Der tatsächliche Aufnahmepfad der Kamera kann also ermittelt werden, wobei die Zentren der Aufnahmebereiche der Aufnahmen graphisch dargestellt und verbunden werden. Dies ermöglicht dem Benutzer auf eine einfache Art und Weise die Abweichung zwischen dem tatsächlichen Aufnahmepfad der Kamera und dem geplanten ersten Aufnahmepfad mitzuverfolgen.

Zur Verbesserung der Registrierung kann die ermittelte Kieferkurve aus der okklusalen Aufnahme, die die tatsächlichen Zahnzentren der Zähne verbindet, für eine so genannte Ausreißerverwerfung verwendet werden.

Bei der okklusalen Vermessung können beispielsweise Strukturen die außerhalb eines festgelegten Abstandes relativ zur Kieferkurve angeordnet sind ausgeblendet werden.

Bei der lingualen Vermessung werden lediglich Strukturen bis zur Kieferkurve berücksichtigt und Strukturen, die in bukkaler Richtung hinter der Kieferkurve angeordnet sind, ausgeblendet.

Bei der bukkalen Vermessung werden lediglich Strukturen bis zur Kieferkurve berücksichtigt und Strukturen, die in lingualer Richtung hinter der Kieferkurve angeordnet sind, ausgeblendet.

Dadurch werden also störende Objekte, wie Zunge des Patienten oder Finger des behandelnden Zahnarztes, ausgeblendet, da sie weiter weg von der Kieferkurve angeordnet sind als die aufzunehmende Zahnsubstanz der Zähne.

Vorteilhafterweise kann das Standardkiefermodell in Überlagerung mit den bereits registrierten einzelnen Aufnahmen in Abhängigkeit von einer Aufnahmerichtung der Kamera so geschwenkt werden, dass aufzunehmende Oberflächen des Objekts angezeigt werden, wobei während der okklusalen Vermessung das Standardkiefermodell okklusaler Richtung angezeigt wird, wobei während der lingualen Vermessung das Standardkiefermodell so geschwenkt wird, dass die aufzunehmenden lingualen Oberflächen und die bereits aufgenommenen okklusalen Oberflächen der Zähne sichtbar sind, wobei während der bukkalen Vermessung das Standardkiefermodell so geschwenkt wird, dass die aufzunehmenden bukkalen Oberflächen und die bereits aufgenommenen okklusalen Oberflächen der Zähne sichtbar sind.

Dadurch kann sich der Benutzer während der Vermessung besser am Standardkiefermodell orientieren. Denn die aufzunehmenden Oberflächen werden deutlich angezeigt.

Vorteilhafterweise kann computergestützt automatisch ermittelt werden, in welchen Bereichen die registrierte Gesamtaufnahme des Objekts Lücken aufweist, wobei zur Benutzerführung nacheinander in diesen Bereichen am Standardkiefermodell weitere Kontrollpunkte und/oder weitere Aufnahmenpfade angezeigt werden.

Dadurch erfolgt die automatische Benutzerführung Kontrollpunkt für Kontrollpunkt solange, bis alle Lücken geschlossen sind und die Gesamtaufnahme lückenlos ist.

Vorteilhafterweise können vor der Vermessung bestimmte Bereiche des Objekts, die beispielsweise eine Präparation enthalten, am Standardkiefermodell festgelegt werden, die vollständig vermessen werden sollen, wobei computergestützt überprüft wird, ob diese Bereiche vollständig vermessen wurden oder Lücken aufweisen, wobei falls diese Bereiche Lücken aufweisen am Standardkiefermodell weitere Kontrollpunkte und/oder weitere Aufnahmenpfade angezeigt werden, um diese Bereiche vollständig zu vermessen.

Dadurch wird gewährleistet, dass beispielsweise für die Planung von Zahnersatzteilen wesentliche Bereiche des Objekts vollständig und lückenlos vermessen werden.

### Kurzbeschreibung der Zeichnungen

Die Erfindung wird anhand der Zeichnungen erläutert. Es zeigt, die
- Fig. 1: eine Skizze zur Verdeutlichung des vorliegenden Verfahrens zur Durchführung einer optischen dreidimensionalen Aufnahme; die
- Fig. 2: eine Skizze eines Standardkiefermodells eines Oberkiefers bei einer okklusalen Vermessung; die
- Fig. 3: eine Skizze eines Standardkiefermodells eines Oberkiefers bei einer lingualen Vermessung; die
- Fig. 4: eine Skizze eines Standardkiefermodells eines Oberkiefers bei einem ersten Schritt einer bukkalen Vermessung; die
- Fig. 5: eine Skizze eines Standardkiefermodells eines Oberkiefers bei einem zweiten Schritt einer bukkalen Vermessung; die
- Fig. 6: eine Skizze zur Verdeutlichung von mehreren Streifen-Aufnahmesequenzen; die
- Fig. 7: eine Skizze zur Verdeutlichung einer Verknüpfung der unterschiedlichen Cluster; die
- Fig. 8: eine Skizze zur Verdeutlichung einer Aufbissregistrierung.

### Ausführungsbeispiele

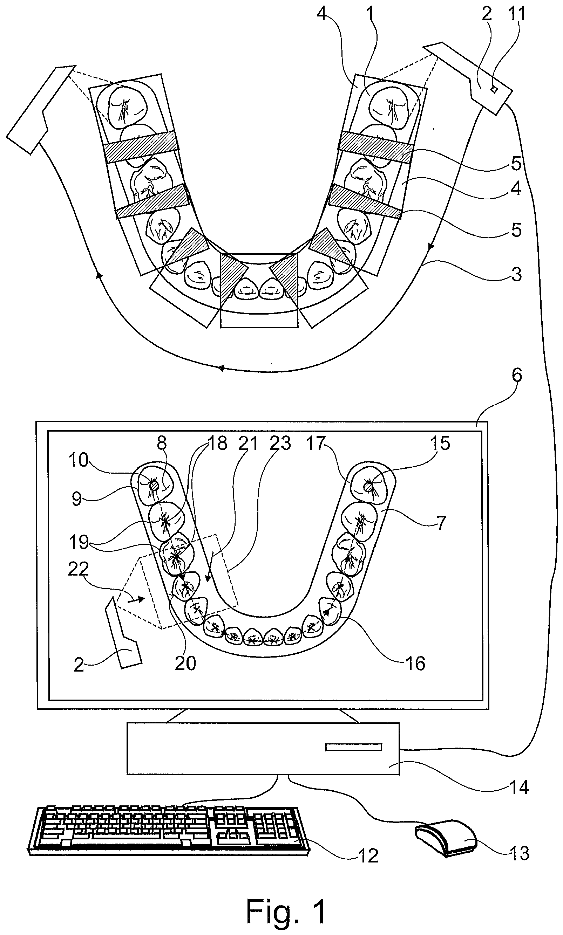

Die Fig. 1 zeigt eine Skizze zur Verdeutlichung des vorliegenden Verfahrens zur Durchführung einer optischen dreidimensionalen Aufnahme eines zu vermessenden dentalen Objekts 1, wie eines Unterkiefers, mittels einer handgehaltenen dentalen Kamera 2, die um das dentale Objekt entlang einer Bewegungsbahn 3 um das dentale Objekt 1 geschwenkt wird. Die dreidimensionalen optischen Aufnahmen 4, die in Form von Rechtecken dargestellt sind, werden mittels der dentalen Kamera 2 vermessen, die während der Vermessung entlang der Bewegungsbahn 3 um das Objekt 1 geschwenkt wird. Die Kamera 2 ist eine handgehaltene Kamera, die unter Verwendung eines Streifenprojektionsverfahrens funktioniert. Die Aufnahmen 4 werden unter Verwendung der Überlappungsbereiche 5, die gestrichelt dargestellt sind, zueinander registriert und dadurch eine Gesamtaufnahme des Objekts 1 erzeugt. Vor der Durchführung der Vermessung wird mittels einer Anzeigevorrichtung 6, wie eines Monitors ein dreidimensionales Standardkiefermodell 7 angezeigt, das in seinen Abmessungen einem durchschnittlichen Kieferbogen entspricht. Dabei wird auf dem Standardkiefermodell des Unterkiefers unten rechts am linken Ende des Standardkiefermodells 7 in der Mitte einer Okklusalfläche 8 des hinteren Backenzahns 9 mit der FDI-Nummer 38 unten links ein erster Kontrollpunkt 10 angezeigt. Der Kontrollpunkt 10 wird schematisch als ein schwarzer Kreis dargestellt. Anschließend bewegt der Benutzer die dentale Kamera in den Bereich des Backenzahns 9, so dass die Kamera den ersten Kontrollpunkt 10 aufnimmt. Die Kamera 2 wird dann für eine festgelegte Zeitdauer über dem ersten Kontrollpunkt stillgehalten, bis ein akustisches, visuelles und/oder haptisches Signal als Rückmeldung erfolgt und damit die Position des ersten Kontrollpunktes bestätigt wird. Das Bestätigen des ersten Kontrollpunktes 10 kann auch durch das Betätigen einer Taste 11 an der Kamera 2 erfolgen.

Alternativ dazu kann das Bestätigen des Kontrollpunktes 10 auch mittels der Eingabemittel, wie einer Tastatur 12 und einer Maus 13, die an einen Computer 14 angeschlossen sind, erfolgen.

Zusätzlich zum ersten Kontrollpunkt wird ein zweiter Kontrollpunkt 15 sowie ein erster Aufnahmepfad 16 angezeigt. Der zweite Kontrollpunkt ist dabei am gegenüberliegenden Ende des Kieferbogens am hinteren Backenzahn 17 mit der FDI-Nummer 48 angeordnet. Der Aufnahmepfad 16 läuft dabei durch die Zahnzentren 18 der einzelnen Zähne 19 des Standardkiefermodells 7. Der angezeigte Aufnahmepfad dient als eine Benutzerführung für den Benutzer um anzuzeigen, welche Bereiche des Objekts 1 vermessen werden sollen.

Zur Orientierung kann das Standardkiefermodell 7 so geschwenkt werden, dass aufzunehmende bukkale Oberflächen 20 eines Aufnahmebereiches 23, der gestrichelt dargestellt ist, im Vordergrund angezeigt werden, wobei auch die bereits vermessenen okklusalen Flächen des Objekts sichtbar sind.

Dadurch wird also die Blickrichtung auf Standardkiefermodell 7 simultan zur Bewegung der Kamera 2 während der Vermesseung verändert, wo dass der Benutzer, wie ein Zahnarzt, sich leichter in der Zahnsituation orientieren kann.

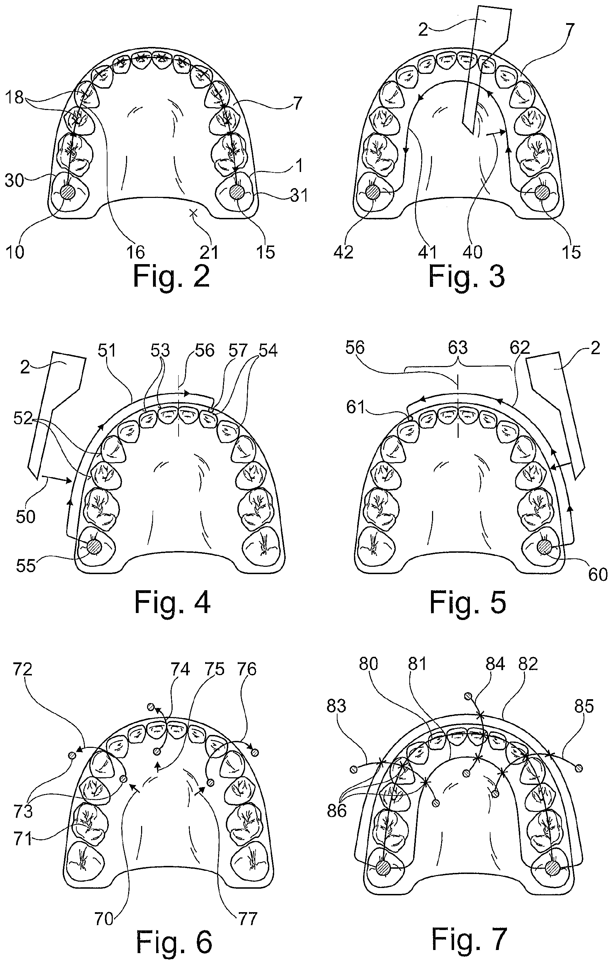

Die Fig. 2 zeigt eine Skizze eines Standardkiefermodells 7 eines Oberkiefers wobei der erste Aufnahmepfad 16 ausgehend von einem ersten Kontrollpunkt 10 oben rechts an einem ersten Backenzahn 30 mit der FDI-Nummer 37 bis zum zweiten Kontrollpunkt 15 am gegenüberliegenden Ende des Kieferbogens am zweiten Backenzahn 31 mit der FDI-Nummer 47 verläuft. Der Aufnahmepfad 16 verläuft dabei durch die Zahnzentren 18 der einzelnen Zähne des Standardkiefermodells 7. Die dentale Kamera 2 wird also so bewegt, dass ein Zentrum der Aufnahmen 4 mit dem Aufnahmepfad 16 übereinstimmt. Auf diese Weise erfolgt also eine okklusale Vermessung aus der okklusalen Richtung 21 des Oberkiefers.

Die Fig. 3 zeigt eine Skizze zur Verdeutlichung einer lingualen Vermessung des Oberkiefers aus einer lingualen bzw. oralen Richtung 40, die durch einen Pfeil dargestellt ist. Die Kamera 2 wird dann relativ zum Objekt 1 so positioniert, dass die Aufnahme aus dieser Richtung 40 ermöglicht wird, wie in Fig. 3 angedeutet. Die linguale Vermessung erfolgt entlang eines zweiten Aufnahmepfades 41 ausgehend vom zweiten Kontrollpunkt 15 zu einem dritten Kontrollpunkt 42 hin. Bei der lingualen Vermessung werden also die innenliegenden Zahnoberflächen des Oberkiefers vermessen.

Die Fig. 4 zeigt eine Skizze zur Verdeutlichung einer buckalen Vermessung aus einer bukkalen Richtung 50, wobei eine Kamera 2 um den Kiefer so geschwenkt wird, dass die buckalen Zahnoberflächen 52 und die labialen Zahnoberflächen 53 vermessen werden. Die Zähne 54 werden also im ersten Schritt nicht vermessen. Der dritte Aufnahmepfad 51 verläuft dabei ausgehend von einem vierten Kontrollpunkt 55 am Backenzahn mit der FDI-Nummer 37 über eine Mitte 56 des Kieferbogens bis zu einem fünften Kontrollpunkt 57. Die Position des vierten Kontrollpunkts 55 kann dabei der Position des Kontrollpunktes 42 und des Kontrollpunktes 10 entsprechen.

In Fig. 5 zeigt eine zweite bukkale Vermessung ausgehend von einem sechsten Kontrollpunkt 60 über die Mitte 56 des Kieferbogens zu einem siebten Kontrollpunkt 61 entlang eines vierten Aufnahmepfades 62. Ein erstes Cluster aus der ersten bukkalen Vermessung aus Fig. 4 und zweites Cluster aus der zweiten bukkalen Vermessung aus Fig. 5 werden dann unter Verwendung eines gemeinsamen Überlappungsbereichs 63 in der Mitte des Kieferbogens zueinander registriert.

Die Fig. 6 zeigt eine Skizze zur Verdeutlichung einer ersten Streifen-Aufnahmesequenz in bukkaler Richtung 70 senkrecht zu einer Kieferkurve 71 des zu vermessenden Kieferbogens entlang eines fünften Aufnahmepfades 72 zwischen den Kontrollpunkten 73. Der fünfte Aufnahmepfad 72 verläuft dabei im Bereich eines Backenzahns mit der FDI-Nummer 14. Zusätzlich wird eine zweite Streifen-Aufnahmesequenz entlang eines sechsten Aufnahmepfades 74 im Bereich des Schneidezahns mit der FDI-Nummer 11 in labialer Richtung 75 sowie eine dritte Streifen-Aufnahmesequenz entlang des siebten Aufnahmepfads 76 im Bereich des Backenzahns mit der FDI-Nummer 24 in bukkaler Richtung 77 durchgeführt.

Die Fig. 7 zeigt eine Skizze die verdeutlichen soll, dass ein erstes Cluster 80 aus der okklusalen Vermessung in Fig. 2, ein zweites Cluster 81 aus der lingualen Vermessung in Fig. 3 und ein drittes Cluster 82 aus der bukkalen Vermessung in Fig. 4 und Fig. 5 durch das vierte Cluster 83 der ersten Streifen-Aufnahmesequenz aus Fig. 6 sowie durch das fünfte Cluster 84 der zweiten Streifen-Aufnahmesequenz und das sechste Cluster 85 der dritten Streifen-Aufnahmesequenz miteinander verknüpft werden. Die Verknüpfungspunkte 86 sind durch die Kreuze angedeutet.

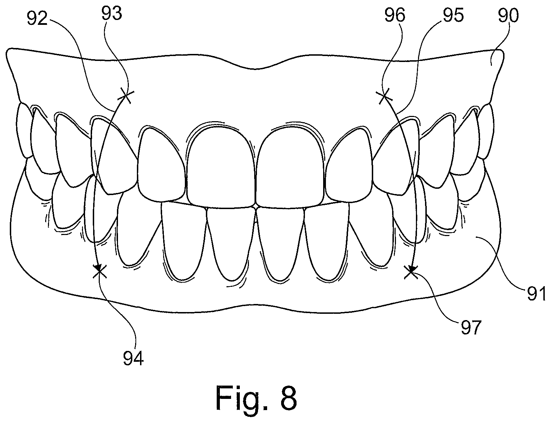

Die Fig. 8 zeigt eine Skizze zur Verdeutlichung einer Aufbissregistrierung, wobei ein erstes dreidimensionales Modell 90 des Oberkiefers relativ zu einem zweiten dreidimensionalen Modell 91 des Unterkiefers registriert wird. Dabei wird eine erste bukkale Aufnahmesequenz entlang eines Aufnahmepfades 92 zwischen den Kontrollpunkten 93 und 94 sowie eine zweite bukkale Aufnahmesequenz entlang des Aufnahmepfades 95 zwischen einem Kontrollpunkt 96 und einem Kontrollpunkt 97 durchgeführt. Die erste bukkale Aufnahmesequenz verläuft dabei im Bereich der Zähne mit den FDI-Nummern 14 und 44. Die zweite bukkale Aufnahmesequenz verläuft im Bereich der Zähne mit den FDI-Nummern 24 und 34.

### Bezugszeichen

- 1: Objekt
- 2: Kamera
- 3: Bewegungsbahn
- 4: Aufnahme
- 5: Überlappungsbereich
- 6: Anzeigevorrichtung
- 7: Standardkiefermodell
- 8: Okklusalfläche
- 9: Backenzahn
- 10: Kontrollpunkt
- 11: Taste
- 12: Tastatur
- 13: Maus
- 14: Computer
- 15: zweiter Kontrollpunkt
- 16: erster Aufahmepfad
- 17: hinterer Backenzahn
- 18: Zahnzentren
- 19: Zähne
- 20: bukkale Oberflächen
- 21: okklusale Richtung
- 22: bukkale Richtung
- 23: Aufnahmebereich
- 30: erster Backenzahn
- 31: zweiter Backenzahn
- 40: Richtung
- 41: Aufnahmepfad
- 50: bukkale Richtung
- 51: dritter Aufnahmepfad
- 52: bukkale Zahnoberfläche
- 53: labiale Zahnoberfläche
- 54: Zähne
- 55: vierter Kontrollpunkt
- 56: Mitte
- 57: fünfter Kontrollpunkt
- 60: sechster Kontrollpunkt
- 61: siebter Kontrollpunkt
- 62: vierter Aufnahmepfad
- 63: Überlappungsbereich
- 70: Richtung
- 71: Kieferkurve
- 72: fünfte Aufnahmepfad
- 73: Kontrollpunkt
- 74: sechster Aufnahmepfad
- 75: siebter Aufnahmepfad
- 76: Richtung
- 80: erster Cluster
- 81: zweiter Cluster
- 82: dritter Cluster
- 83: vierter Cluster
- 84: fünfter Cluster
- 85: sechster Cluster
- 86: Verknüpfungspunkt
- 90: erstes dreidimensionales Modell
- 91: zweites dreidimensionales Modell
- 92: Aufnahmepfad
- 93: Kontrollpunkt
- 94: Kontrollpunkt
- 95: Aufnahmepfad
- 96: Kontrollpunkt
- 97: Kontrollpunkt

## Patentansprüche

1. Verfahren zur Durchführung einer optischen dreidimensionalen Aufnahme (4) unter Verwendung einer handgehaltenen dentalen Kamera (2), wobei die Kamera (2) während der Vermessung mehrere einzelne dreidimensionale optische Aufnahmen (4) nacheinander mit einer festgelegten Frequenz automatisch aufnimmt, wobei die einzelnen dreidimensionalen optischen Aufnahmen (4) zu einer Gesamtaufnahme eines zu vermessenden dentalen Objekts (1) durch ein Registrierungsverfahren zusammengefügt werden, **dadurch gekennzeichnet, dass** vor der Durchführung der Vermessung mittels einer Anzeigevorrichtung (6) ein dreidimensionales Standardkiefermodell (7) angezeigt wird, wobei ein erster Kontrollpunkt (10) am Standardkiefermodell (7) mittels der Anzeigevorrichtung (6) angezeigt wird, wobei die handgehaltene dentale Kamera (2) relativ zum aufzunehmenden Objekt (1) so positioniert wird, dass die Kamera (2) auf einen dem ersten Kontrollpunkt (10) des Standardkiefermodells (7) entsprechenden Punkt auf dem aufzunehmenden Objekt (1) zeigt und einen dem ersten Kontrollpunkt (10) entsprechenden Aufnahmebereich des dentalen Objekts (1) aufnimmt.

2. Verfahren nach Anspruch 1, **dadurch gekennzeichnet, dass** die Position des ersten Kontrollpunktes (10) durch eine Handlung eines Benutzers bestätigt wird.

3. Verfahren nach Anspruch 2, **dadurch gekennzeichnet, dass** der Benutzer die dentale Kamera (2) relativ zum Objekt (1) am ersten Kontrollpunkt (10) für eine festgelegte Zeitdauer still hält bis ein akustisches, visuelles und/oder haptisches Signal als Rückmeldung erfolgt.

4. Verfahren nach Anspruch 2, **dadurch gekennzeichnet, dass** der Benutzer den ersten Kontrollpunkt (10) durch das Betätigen einer Taste (11) an der Kamera (2) bestätigt.

5. Verfahren nach Anspruch 2, **dadurch gekennzeichnet, dass** der Benutzer den ersten Kontrollpunkt (10) durch das Ausführen einer Geste mit der Kamera (2) bestätigt.

6. Verfahren nach Anspruch 2, **dadurch gekennzeichnet, dass** der Benutzer den ersten Kontrollpunkt (10) durch einen Sprachbefehl bestätigt, indem der Sprachbefehl in einer Audioaufnahme aufgezeichnet wird und mittels einer Spracherkennung erkannt wird.

7. Verfahren nach Anspruch 2, **dadurch gekennzeichnet, dass** der Benutzer den ersten Kontrollpunkt (10) durch das Betätigen eines Eingabemittels, wie einer Maus (13) oder eine Tastatur (12), bestätigt.

8. Verfahren nach einem der Ansprüche 1 bis 7, **dadurch gekennzeichnet, dass** das dentale Objekt (1) ein gesamter Oberkiefer und/oder ein Unterkiefer ist.

9. Verfahren nach einem der Ansprüche 1 bis 8, **dadurch gekennzeichnet, dass** der erste Kontrollpunkt (10) in der Mitte (56) einer Okklusalfläche (8) eines Backenzahns (9) angeordnet ist, der sich an einem linken Ende oder an einem rechten Ende des Standardkiefermodells (7) befindet.

10. Verfahren nach einem der Ansprüche 1 bis 9, **dadurch gekennzeichnet, dass** der Benutzer die Lage des festgelegten ersten Kontrollpunktes (10) unter Verwendung eines Eingabemittels relativ zum Standardkiefermodell (7) verschiebt und neu festlegt.

11. Verfahren nach einem der Ansprüche 1 bis 10, **dadurch gekennzeichnet, dass** zusätzlich zum ersten Kontrollpunkt (10) an einem gegenüberliegenden Ende des Kiefers als Objekt (1) ein zweiter Kontrollpunkt (15) festgelegt wird und mittels der Anzeigevorrichtung (6) angezeigt wird, wobei zwischen dem ersten Kontrollpunkt (10) und dem zweiten Kontrollpunkt (15) ein erster Aufnahmepfad (16) festgelegt wird, der mittels der Anzeigevorrichtung (6) am Standardkiefermodell (7) angezeigt wird.

12. Verfahren nach Anspruch 11, **dadurch gekennzeichnet, dass** eine okklusale Vermessung aus einer okklusalen Richtung (21) relativ zum Kiefer als Objekt (1) durchgeführt wird, wobei die manuell gehaltene Dentalkamera (2) entlang des angezeigten ersten Aufnahmepfads (16) bewegt wird bis der zweite Kontrollpunkt (15) erreicht ist.

13. Verfahren nach einem der Ansprüche 1 bis 12, **dadurch gekennzeichnet, dass** der Benutzer die Position des zweiten Kontrollpunktes (15) durch eine manuelle Handlung bestätigt.

14. Verfahren nach einem der Ansprüche 1 bis 13, **dadurch gekennzeichnet, dass** eine linguale beziehungsweise eine palatinale Vermessung von oral liegenden Zahnflächen des Oberkiefers beziehungsweise des Unterkiefers als Objekt (1) erfolgt, wobei ein dritter Kontrollpunkt am gegenüberliegenden Ende des Kiefers ausgehend vom zweiten Kontrollpunkt (15) festgelegt wird, wobei ein zweiter Aufnahmepfad (41, 92) zwischen dem zweiten Kontrollpunkt (15) und dem dritten Kontrollpunkt festgelegt und mittels der Anzeigevorrichtung (6) angezeigt wird, wobei die handgehaltene Kamera während der Vermessung entlang des zweiten Aufnahmepfads (41) bewegt wird, wobei die Position des dritten Aufnahmepunktes bestätigt wird.

15. Verfahren nach einem der Ansprüche 1 bis 14, **dadurch gekennzeichnet, dass** eine bukkale Vermessung aus einer bukkaler Richtung (50) durchgeführt wird, wobei in einem ersten Schritt eine erste bukkale Vermessung ausgehend von einem vierten Kontrollpunkt (55) an einem Ende des Kieferbogens zur Mitte (56) des Kieferbogens und darüber hinaus bis zu einem fünften Kontrollpunkt (57) entlang eines dritten Aufnahmepfades (51) durchgeführt wird, wobei anschließend in einem zweiten Schritt eine zweite bukkale Vermessung ausgehend von einem sechsten Kontrollpunkt an einem gegenüberliegenden Ende des Kieferbogens zur Mitte (56) des Kieferbogens und darüber hinaus bis zu einem siebten Kontrollpunkt (61) entlang eines vierten Aufnahmepfades (62) durchgeführt wird.

16. Verfahren nach Anspruch 15, **dadurch gekennzeichnet, dass** ein erstes Cluster aus der ersten bukkalen Vermessung und ein zweites Cluster aus der zweiten bukkalen Vermessung unter Verwendung eines gemeinsamen Überlappungsbereichs (63) in der Mitte (56) des Kieferbogens zueinander registriert werden.

17. Verfahren nach einem der Ansprüche 1 bis 16, **dadurch gekennzeichnet, dass** mindestens eine Streifen-Aufnahmesequenz in bukkaler bzw. labialer Richtung senkrecht zu einer Kieferkurve des zu vermessenden Kieferbogens entlang eines fünften Aufnahmepfades (72), der vor der Vermessung dieser Streifen- Aufnahmesequenz mittels der Anzeigevorrichtung (6) angezeigt wird, durchgeführt wird, wobei bereits erzeugte Cluster aus der okklusalen Vermessung, der lingualen Vermessung und/oder der bukkalen Vermessung miteinander verknüpft werden und dadurch die Registrierung verbessert wird.

18. Verfahren nach einem der Ansprüche 1 bis 17, **dadurch gekennzeichnet, dass** eine Aufbissregistrierung durchgeführt wird, wobei ein erstes dreidimensionales Modell des Oberkiefers relativ zu einem zweiten dreidimensionalen Modell des Unterkiefers registriert wird, wobei eine bukkale Aufnahmesequenz in einer Aufbissstellung entlang eines siebten Aufnahmepfades (92) vermessen wird, der mittels der Anzeigevorrichtung (6) zwischen entsprechenden Kontrollpunkten (93, 94) angezeigt wird.

19. Verfahren nach einem der Ansprüche 1 bis 18, **dadurch gekennzeichnet, dass** ein tatsächlicher Aufnahmepfad der Kamera ermittelt wird, der durch die Projektionen der Zentren der einzelnen Aufnahmen entlang einer Kameraachse auf einer Oberfläche des Objekts gebildet wird, wobei der tatsächliche Aufnahmepfad der Kamera mittels der Anzeigevorrichtung angezeigt wird.

20. Verfahren nach Anspruch 19, **dadurch gekennzeichnet, dass** während der Vermessung eine Abweichung zwischen den tatsächlichen Aufnahmepfad und dem geplanten Aufnahmepfad ermittelt wird.

21. Verfahren nach Anspruch 20, **dadurch gekennzeichnet, dass** falls diese Abweichung einen festgelegten Schwellenwert überschreitet, eine Fehlermeldung mittels der Anzeigevorrichtung angezeigt wird und daraufhin der Benutzer aufgefordert wird die Vermessung an einem neuen Kontrollpunkt fortzusetzen.

22. Verfahren nach einem der Ansprüche 1 bis 21, **dadurch gekennzeichnet, dass** das Standardkiefermodell in Überlagerung mit den bereits registrierten einzelnen Aufnahmen in Abhängigkeit von einer Aufnahmerichtung der Kamera so geschwenkt wird, dass aufzunehmende Oberflächen des Objekts angezeigt werden, wobei während der okklusalen Vermessung das Standardkiefermodell okklusaler Richtung angezeigt wird, wobei während der lingualen Vermessung das Standardkiefermodell so geschwenkt wird, dass die aufzunehmenden lingualen Oberflächen und die bereits aufgenommenen okklusalen Oberflächen der Zähne sichtbar sind, wobei während der bukkalen Vermessung das Standardkiefermodell so geschwenkt wird, dass die aufzunehmenden bukkalen Oberflächen und die bereits aufgenommenen okklusalen Oberflächen der Zähne sichtbar sind.

23. Verfahren nach einem der Ansprüche 1 bis 22, **dadurch gekennzeichnet, dass** computergestützt automatisch ermittelt wird in welchen Bereichen die registrierte Gesamtaufnahme des Objekts Lücken aufweist, wobei zur Benutzerführung nacheinander in diesen Bereichen am Standardkiefermodell weitere Kontrollpunkte und/oder weitere Aufnahmenpfade angezeigt werden.

24. Verfahren nach einem der Ansprüche 1 bis 23, **dadurch gekennzeichnet, dass** vor der Vermessung bestimmte Bereiche des Objekts, wie eine Präparation, am Standardkiefermodell festgelegt werden, die vollständig vermessen werden sollen, wobei computergestützt überprüft wird, ob diese Bereiche vollständig vermessen wurden oder Lücken aufweisen, wobei falls diese Bereiche Lücken aufweisen am Standardkiefermodell weitere Kontrollpunkte und/oder weitere Aufnahmenpfade angezeigt werden, um diese Bereiche vollständig zu vermessen.

## Claims

1. A method for performing an optical three-dimensional recording (4) using a hand-held dental camera (2), the camera (2) automatically taking a plurality of individual three-dimensional optical recordings (4) in succession at a defined frequency during the measurement, the individual three-dimensional optical recordings (4) being combined using a registration method into an overall recording of a dental object (1) to be measured, **characterized in that** a three-dimensional standard jaw model (7) is displayed by means of a display device (6) before the measurement is carried out, a first control point (10) on the standard jaw model (7) being displayed by means of the display device (6), the hand-held dental camera (2) being positioned relative to the object (1) to be recorded in such a way that the camera (2) points to a point on the object (1) to be recorded corresponding to the first control point (10) of the standard jaw model (7) and records a recording region of the dental object (1) corresponding to the first control point (10).

2. The method according to claim 1, **characterised in that** the position of the first control point (10) is confirmed by an action by a user.

3. The method according to claim 2, **characterised in that** the user holds the dental camera (2) still relative to the object (1) at the first control point (10) for a fixed period of time until an acoustic, visual and/or haptic signal is issued as feedback.

4. The method according to claim 2, **characterised in that** the user confirms the first control point (10) by pressing a button (11) on the camera (2).

5. The method according to claim 2, **characterised in that** the user confirms the first control point (10) by executing a gesture with the camera (2).

6. The method according to claim 2, **characterised in that** the user confirms the first control point (10) by means of a voice command, by the voice command being recorded in an audio recording and being recognised by means of a voice recognition system.

7. The method according to claim 2, **characterised in that** the user confirms the first control point (10) by actuating an input means such as a mouse (13) or a keyboard (12).

8. The method according to one of claims 1 to 7, **characterised in that** the dental object (1) is an entire upper jaw and/or a lower jaw.

9. The method according to one of claims 1 to 8, **characterised in that** the first control point (10) is arranged in the centre (56) of an occlusal surface (8) of a molar tooth (9), which is located at a left-hand end or at a right-hand end of the standard jaw model (7).

10. The method according to one of claims 1 to 9, **characterised in that** the user shifts and redefines the position of the defined first control point (10) relative to the standard jaw model (7) using an input means.

11. The method according to one of claims 1 to 10, **characterised in that**, in addition to the first control point (10), a second control point (15) is defined as an object (1) at an opposite end of the jaw and is displayed by means of the display device (6), wherein between the first control point (10) and the second control point (15) a first recording path (16) is defined, which is displayed on the standard jaw model (7) by means of the display device (6).

12. The method according to claim 11, **characterised in that** an occlusal measurement is carried out from an occlusal direction (21) relative to the jaw as an object (1), wherein the manually held dental camera (2) is moved along the displayed first recording path (16) until the second control point (15) is reached.

13. The method according to one of claims 1 to 12, **characterised in that** the user confirms the position of the second control point (15) by a manual action.

14. The method according to one of claims 1 to 13, **characterised in that** a lingual or a palatal measurement of oral tooth surfaces of the upper jaw or lower jaw as an object (1) is carried out, a third control point at the opposite end of the jaw being determined starting from the second control point (15), a second recording path (41, 92) being defined between the second control point (15) and the third control point and being displayed by means of the display device (6), wherein the hand-held camera is moved along the second recording path (41) during the measurement, the position of the third recording point being confirmed.

15. The method according to one of claims 1 to 14, **characterised in that** a buccal measurement is carried out from a buccal direction (50), wherein in a first step a first buccal measurement is carried out starting from a fourth control point (55) at one end of the jaw arch to the centre (56) of the jaw arch and beyond up to a fifth control point (57) along a third recording path (51), wherein in a second step a second buccal measurement is subsequently carried out starting from a sixth control point at an opposite end of the jaw arch to the centre (56) of the jaw arch and beyond up to a seventh control point (61) along a fourth recording path (62).

16. The method according to claim 15, **characterised in that** a first cluster from the first buccal measurement and a second cluster from the second buccal measurement are registered relative to one another using a common overlap region (63) in the middle (56) of the jaw arch.

17. The method according to one of claims 1 to 16, **characterised in that** at least one strip recording sequence is carried out in the buccal or labial direction perpendicularly to a jaw curve of the jaw arch to be measured along a fifth recording path (72), which before the measurement of this strip recording sequence is displayed by means of the display device (6), wherein previously generated clusters from the occlusal measurement, the lingual measurement and/or the buccal measurement are linked to one another, thereby improving the registration.

18. The method according to one of claims 1 to 17, **characterised in that** a bite registration is carried out, wherein a first three-dimensional model of the upper jaw is registered relative to a second three-dimensional model of the lower jaw, a buccal recording sequence in a bite-down position being measured along a seventh recording path (92), which is displayed by means of the display device (6) between corresponding control points (93, 94).

19. The method according to one of claims 1 to 18, **characterised in that** an actual recording path of the camera is determined and is formed by the projections of the centres of the individual recordings along a camera axis on a surface of the object, the actual recording path of the camera being displayed by means of the display device.

20. The method according to claim 19, **characterised in that** a deviation between the actual recording path and the planned recording path is determined during the measurement.

21. The method according to claim 20, **characterised in that** if this deviation exceeds a defined threshold value, an error message is displayed by means of the display device and the user is then prompted to continue the measurement at a new control point.

22. The method according to one of claims 1 to 21, **characterised in that** the standard jaw model, being superimposed with the already registered individual recordings, is pivoted depending on a recording direction of the camera such that object surfaces to be recorded are displayed, the standard jaw model being displayed from an occlusal direction during the occlusal measurement, the standard jaw model being pivoted during the lingual measurement such that the lingual surfaces to be recorded and the occlusal surfaces of the teeth that have already been recorded are visible, wherein during the buccal measurement the standard jaw model is pivoted such that the buccal surfaces to be recorded and the already recorded occlusal surfaces of the teeth are visible.

23. The method according to one of claims 1 to 22, **characterised in that** the regions in which the registered overall image of the object has gaps are determined automatically by computer, wherein, to provide user guidance, further control points and/or additional recording paths are displayed successively in these regions on the standard jaw model.

24. The method according to one of claims 1 to 23, **characterised in that** prior to the measurement certain regions of the object which are to be measured completely, such as a preparation, are defined on the standard jaw model, it being checked by computer whether these regions have been completely measured or have gaps, wherein if these regions have gaps, further control points and/or further recording paths are displayed on the standard jaw model in order to measure these regions completely.

## Revendications

1. Procédé de réalisation d'un cliché optique tridimensionnel (4) à l'aide d'une caméra dentaire portative (2), la caméra (2) prenant automatiquement plusieurs clichés optiques tridimensionnels individuels (4) successivement à une fréquence fixe pendant la mesure, les clichés optiques tridimensionnels individuels (4) étant combinés en un cliché global d'un objet dentaire (1) à mesurer par une méthode d'enregistrement, **caractérisé en ce qu'**un modèle de mâchoire standard tridimensionnel (7) est affiché au moyen d'un dispositif d'affichage (6) avant d'effectuer la mesure, un premier point de contrôle (10) étant affiché sur le modèle de mâchoire standard (7) au moyen du dispositif d'affichage (6), la caméra dentaire portative (2) étant positionnée par rapport à l'objet (1) à photographier de telle sorte que la caméra (2) vise un point correspondant au premier point de contrôle (10) du modèle de mâchoire standard (7) sur l'objet (1) à photographier et prend un cliché d'une zone à photographier de l'objet dentaire (1) correspondant au premier point de contrôle (10).

2. Procédé selon la revendication 1, **caractérisé en ce que** la position du premier point de contrôle (10) est confirmée par une action d'un utilisateur.

3. Procédé selon la revendication 2, **caractérisé en ce que** l'utilisateur maintient la caméra dentaire (2) immobile par rapport à l'objet (1) au premier point de contrôle (10) pendant une période de temps fixe jusqu'à ce qu'un signal acoustique, visuel et/ou haptique soit émis à titre de confirmation.

4. Procédé selon la revendication 2, **caractérisé en ce que** l'utilisateur confirme le premier point de contrôle (10) en appuyant sur un bouton (11) sur la caméra (2).

5. Procédé selon la revendication 2, **caractérisé en ce que** l'utilisateur confirme le premier point de contrôle (10) en exécutant un geste avec la caméra (2).

6. Procédé selon la revendication 2, **caractérisé en ce que** l'utilisateur confirme le premier point de contrôle (10) au moyen d'une commande vocale, la commande vocale étant enregistrée dans un enregistrement audio et étant reconnue au moyen d'une reconnaissance vocale.

7. Procédé selon la revendication 2, **caractérisé en ce que** l'utilisateur confirme le premier point de contrôle (10) en actionnant un moyen d'entrée tel qu'une souris (13) ou un clavier (12).

8. Procédé selon l'une des revendications 1 à 7, **caractérisé en ce que** l'objet dentaire (1) est une mâchoire supérieure entière et/ou une mâchoire inférieure.

9. Procédé selon l'une des revendications 1 à 8, **caractérisé en ce que** le premier point de contrôle (10) se trouve au milieu (56) d'une surface occlusale (8) d'une molaire (9), qui est située à une extrémité gauche ou à une extrémité droite du modèle de mâchoire standard (7).

10. Procédé selon l'une des revendications 1 à 9, **caractérisé en ce que** l'utilisateur décale et redéfinit la position du premier point de contrôle défini (10) par rapport au modèle de mâchoire standard (7) à l'aide d'un moyen d'entrée.

11. Procédé selon l'une des revendications 1 à 10, **caractérisé en ce que**, outre le premier point de contrôle (10), un deuxième point de contrôle (15) est défini à une extrémité opposée de la mâchoire en tant qu'objet (1) et est affiché au moyen du dispositif d'affichage (6), dans lequel entre le premier point de contrôle (10) et le deuxième point de contrôle (15), un premier trajet de prises de vue (16) est défini, qui est affiché sur le modèle de mâchoire standard (7) au moyen du dispositif d'affichage (6).

12. Procédé selon la revendication 11, **caractérisé en ce qu'**une mesure occlusale est effectuée à partir d'une direction occlusale (21) par rapport à la mâchoire en tant qu'objet (1), dans lequel la caméra dentaire portative (2) est déplacée le long du premier trajet de prises de vue affiché (16) jusqu'à ce que le deuxième point de contrôle (15) soit atteint.

13. Procédé selon l'une des revendications 1 à 12, **caractérisé en ce que** l'utilisateur confirme la position du deuxième point de contrôle (15) par une action manuelle.

14. Procédé selon l'une des revendications 1 à 13, **caractérisé en ce qu'**une mesure linguale ou palatine des surfaces dentaires buccales de la mâchoire supérieure ou inférieure en tant qu'objet (1) est réalisée, un troisième point de contrôle étant défini à l'extrémité opposée de la mâchoire à partir du deuxième point de contrôle (15), un deuxième trajet de prises de vue (41, 92) étant défini entre le deuxième point de contrôle (15) et le troisième point de contrôle et affiché au moyen du dispositif d'affichage (6), la caméra portative se déplaçant le long du deuxième trajet de prises de vue (41) pendant la mesure, la position du troisième point de prises de vue étant confirmée.

15. Procédé selon l'une des revendications 1 à 14, **caractérisé en ce qu'**une mesure buccale est effectuée à partir d'une direction buccale (50), dans lequel, dans une première étape, une première mesure buccale est réalisée à partir d'un quatrième point de contrôle (55) à une extrémité de l'arcade de la mâchoire jusqu'au centre (56) de l'arcade de la mâchoire et au-delà jusqu'à un cinquième point de contrôle (57) le long d'un troisième trajet de prises de vue (51), ensuite dans une deuxième étape, une deuxième mesure buccale est réalisée à partir d'un sixième point de contrôle à une extrémité opposée de l'arcade de la mâchoire jusqu'au centre (56) de l'arcade de la mâchoire et au-delà jusqu'à un septième point de contrôle (61) le long d'un quatrième trajet de prises de vue (62).

16. Procédé selon la revendication 15, **caractérisé en ce qu'**un premier groupe constitué de la première mesure buccale et un deuxième groupe constitué de la deuxième mesure buccale sont enregistrés l'un avec l'autre en utilisant une zone de chevauchement commune (63) au milieu (56) de l'arcade de la mâchoire.

17. Procédé selon l'une des revendications 1 à 16, **caractérisé en ce qu'**au moins une séquence de prises de vue de bande dans la direction buccale ou labiale perpendiculaire à une courbe de mâchoire de l'arcade de la mâchoire à mesurer le long d'un cinquième trajet de prises de vue (72), qui avant la mesure de cette séquence de prises de vue de bande est affiché au moyen du dispositif d'affichage (6), est exécutée, dans lequel des groupes déjà générés à partir de la mesure occlusale, de la mesure linguale et/ou de la mesure buccale sont reliés les uns aux autres et ainsi l'enregistrement est amélioré.

18. Procédé selon l'une des revendications 1 à 17, **caractérisé en ce qu'**un enregistrement occlusal est effectué, un premier modèle tridimensionnel de la mâchoire supérieure étant enregistré par rapport à un deuxième modèle tridimensionnel de la mâchoire inférieure, une séquence de prises de vue buccales en position occlusale le long d'un septième trajet de prises de vue (92) étant mesurée, laquelle est affichée au moyen du dispositif d'affichage (6) entre les points de contrôle correspondants (93, 94).

19. Procédé selon l'une des revendications 1 à 18, **caractérisé en ce qu'**un trajet effectif de prises de vue de la caméra est déterminé, qui est formé par les projections des centres des prises de vue individuelles le long d'un axe de caméra sur une surface de l'objet, le trajet effectif de prises de vue de la caméra étant affiché au moyen du dispositif d'affichage.

20. Procédé selon la revendication 19, **caractérisé en ce qu'**un écart entre le trajet effectif de prises de vue et le trajet prévu de prises de vue est déterminé pendant la mesure.

21. Procédé selon la revendication 20, **caractérisé en ce que** si cet écart dépasse une valeur de seuil définie, un message d'erreur est affiché au moyen du dispositif d'affichage et l'utilisateur est alors invité à poursuivre la mesure à un nouveau point de contrôle.

22. Procédé selon l'une des revendications 1 à 21, **caractérisé en ce que** le modèle de mâchoire standard est pivoté en superposition avec les prises de vue individuelles déjà enregistrées en fonction d'une direction de prises de vue de la caméra de sorte que les surfaces de l'objet à photographier sont affichées, le modèle de mâchoire standard étant affiché en direction occlusale lors de la mesure occlusale, le modèle de mâchoire standard étant pivoté pendant la mesure linguale afin que les surfaces linguales à photographier et les surfaces occlusales des dents qui ont déjà été photographiées soient visibles, et, pendant la mesure buccale, le modèle de mâchoire standard est pivoté de sorte que les surfaces buccales à photographier et les surfaces occlusales déjà photographiées des dents soient visibles.

23. Procédé selon l'une des revendications 1 à 22, **caractérisé en ce qu'**une assistance par ordinateur détermine automatiquement dans quelles zones l'image globale enregistrée de l'objet présente des lacunes, d'autres points de contrôle et/ou d'autres trajets de prises de vue étant affichés successivement dans ces zones sur le modèle de mâchoire standard en vue de guider l'utilisateur.

24. Procédé selon l'une des revendications 1 à 23, **caractérisé en ce qu'**avant la mesure, certaines zones de l'objet, telles qu'une préparation, sont définies sur le modèle de mâchoire standard, qui doivent être mesurées complètement, une vérification assistée par ordinateur étant réalisée afin de déterminer si ces zones ont été complètement mesurées ou présentent des lacunes, et en cas de lacunes dans ces zones, d'autres points de contrôle et/ou d'autres trajets de prises de vue étant affichés sur le modèle de mâchoire standard afin de mesurer complètement ces zones.
